# EUROPÄISCHE PATENTSCHRIFT

(11) **EP 1 032 333 B1**
(45) Veröffentlichungstag und Bekanntmachung des Hinweises auf die Patenterteilung: **15.09.2004**
(21) Anmeldenummer: 98965576.6
(22) Anmeldetag: 17.11.1998
(51) Int. Cl.: A61F 5/01

(54) **EXOPROTHESE FÜR DAS MENSCHLICHE KNIEGELENK**
EXOPROSTHESIS FOR THE HUMAN KNEE-JOINT
EXOPROTHESE DESTINEE A L'ARTICULATION DU GENOU DE L'HOMME

(30) Priorität: 21.11.1997 DE 19751758
(43) Veröffentlichungstag der Anmeldung: 06.09.2000
(73) Patentinhaber: HJS Gelenk System GmbH, 81925 München (DE)
(72) Erfinder: KUBEIN-MEESENBURG, Dietmar, D-37547 Kreiensen (DE); NÄGERL, Hans, D-37130 Gleichen (DE)
(74) Vertreter: Scheffler, Jörg, Dipl.-Ing.
(86) Internationale Anmeldenummer: PCT/DE1998/003390
(87) Internationale Veröffentlichungsnummer: WO 1999/026564

(56) Entgegenhaltungen:
- EP-A- 0 611 093
- WO-A-88/04543
- US-A- 4 502 472
- US-A- 5 062 858
- US-A- 5 443 444

## Beschreibung

Die vorliegende Erfindung betrifft eine Exoprothese/Orthese für das menschliche Kniegelenk gemäß den Merkmalen des Oberbegriffs des Anspruchs 1, wie sie aus der EP-A-0 611 093 bekannt ist.

Aus der DE-OS 43 09 577 ist bereits eine Exoprothese für das menschliche Kniegelenk bekannt. Das Unterschenkelteil und das Oberschenkelteil sind bei diesem Gelenk durch eine Vier-Gelenkanordnung verbunden, die aus zwei parallel angeordneten Teilgelenken gebildet ist, und zwar einem Medialgelenk und einem Lateralgelenk, die jeweils die Gelenkgeometrie einer Gelenkkette mit zwei Gelenkachsen (dimere Gelenkkette) besitzen. Die Gelenkgeometrie des Medialgelenks ist als eine überschlagene, dimere Gelenkkette ausgebildet, bei der die Gelenkachse des Unterschenkelteils in Richtung auf den Oberschenkelteil gegenüber der Gelenkachse des Oberschenkelteils versetzt ist, und die Gelenkgeometrie des Lateralgelenks ist als gestreckte dimere Kette ausgebildet. Hierbei ist die Gelenkachse des Unterschenkelteils gegenüber der Gelenkachse des Oberschenkelteils in Richtung auf den Unterschenkel versetzt. Die beiden Gelenkachsen des Lateralgelenks und des Medialgelenks sind jeweils mittels eines Koppelglieds miteinander gelenkig verbunden. Bei dieser Ausbildung als Vier-Gelenk ergibt sich eine fest vorgegebene zwangläufige Bewegung des Unterschenkels gegenüber dem Femur bzw. umgekehrt. Bei einer derartigen zwangläufigen Bewegung des Unterschenkelteils gegenüber dem Oberschenkelteil und umgekehrt ist dem jeweiligen Gelenkteil eine bestimmte Rast- bzw. Gangpolbahn zugeordnet. Die Polbahnen (Rastpolbahn-Gangpolbahn) sind die Kurven, auf denen die jeweiligen momentanen Drehpole liegen, die sich bei einer stetigen Bewegung ergeben. Die Berechnung dieser Polbahnen ist allgemein bekannt.

Der vorliegenden Erfindung liegt die Aufgabe zugrunde, ausgehend von der vorbekannten Exoprothese/Orthese, diese durch eine einfachere und platzsparendere Konstruktion zu ersetzen.

Erfindungsgemäß wird diese Aufgabe durch die kennzeichnenden Merkmale des Anspruchs 1 gelöst. Erfindungsgemäß ist somit nicht ein Vier-Gelenk direkt nachgebildet, sondern indirekt, indem die Rast- und Gangpolkurve des Gelenks durch Verzahnungsabschnitte approximiert werden, die dann bedingt durch deren bestimmte Anordnung ineinander abrollen und somit ein gegenläufiges Abrollen der Polbahnen ermöglichen. Die Rast-Polbahn im Referenzsystem des Femurs soll nach unten geöffnet sein und der momentane Drehpol soll sich von der Streckung zur Beuge von hinten nach vorne bewegen. Indem die Verzahnungsbahn der jeweiligen Polbahn angepaßt ist, ergibt sich ein Kurvengetriebe und es wird die Getriebeeigenschaft des natürlichen Knies nahezu exakt erhalten. Durch das kurvenförmige Verzahnungspaar wird eine Zwangführung vorgegeben, die ein Gleiten ausschließt. Um ein Trennen des kurvenförmigen Verzahnungspaares zu verhindern, muß das Oberschenkelteil und das Unterschenkelteil durch eine zweite gelenkige Verbindung (dimere Gelenkkette), bestehend aus der im Oberschenkelteil befindlichen Gelenkachse und der im Unterschenkelteil befindlichen Gelenkachse und einem zwischen diesen befindlichen Koppelglied, verbunden werden. Erfindungsgemäß kann es vorteilhaft sein, wenn die Polbahnen durch Kreisabschnitte approximiert werden, deren Mittelpunkte als Gelenkachse verwandt werden. Erfindungsgemäß ist das den Femur bildende Gelenkteil (Oberschenkelgelenkteil) mit einer Außenverzahnung und das die Tibia bildende Gelenkteil (Unterschenkel-Gelenkteil) mit einer Innenverzahnung versehen , wobei die beiden Verzahnungen dann im Gelenkfunktionsbereich ineinander abrollen. Durch die Ausbildung als Zahnradsegmente ergibt sich eine einfache Herstellungsmöglichkeit. Indem die Mittelpunkte der Zahnräder (approximierte Polbahnen) bzw. die Drehachsen der Zahnräder über ein Koppelglied miteinander verbunden werden, wird der Zwanglauf des Systems bei Krafteinwirkung garantiert, d.h. es wird ein Trennen der verzahnten Gelenkteile verhindert, da eine kraftzugübertragende dimere Kette durch das Koppelglied hinzugefügt wird. Anstelle eines separaten Koppelgliedes kann aber auch eine Kapselung des Gelenks vorgesehen werden, wobei die Kapselwandungen gleichzeitig als kraftübertragende Koppelglieder dienen können.

Weiterhin liegt es im Rahmen der Erfindung, wenn dem erfindungsgemäßen Gelenk mit den verzahnten Gelenkteilen ein hierzu in einer parallelen Ebene angeordnetes zweites entsprechendes Gelenk zugeordnet ist. Hierdurch wird bei demselben Zwanglauf eine größere Stabilität garantiert.

Durch die erfindungsgemäße Ausbildung als Zahngetriebe-Gelenk ergibt sich eine sehr kompakte Ausführung der Exoprothese in Verbindung mit einer preisgünstigen Herstellung.

Anhand des in den beiliegenden Zeichnungen dargestellten Ausführungsbeispiels wird die Erfindung näher erläutert. Es zeigen:
- Fig. 1: eine Seitenansicht einer erfindungsgemäßen Exoprothese/Orthese,
- Fig. 2: eine Seitenansicht einer weiteren Ausführung einer erfindungsgemäßen Exoprothese/Orthese,
- Fig. 3: einen Schnitt entlang der Schnittlinie II-II in Fig. 2,
- Fig. 4: eine Ansicht auf ein menschliches Knie mit daran befestigter erfindungsgemäßer Exoprothese.

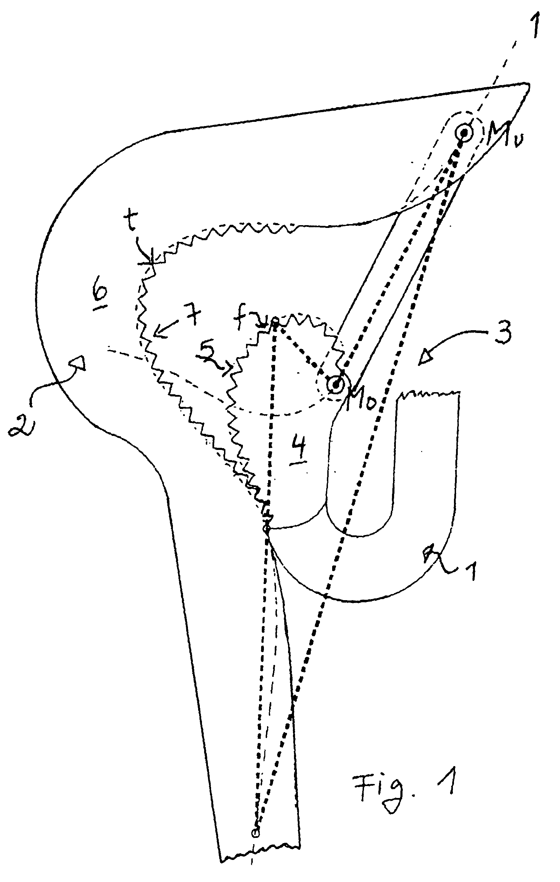

In den Figuren 1 bis 4 sind gleiche Teile mit denselben Bezugsziffern gekennzeichnet.

Wie Fig. 1 zu entnehmen ist, besteht eine erfindungsgemäße Exoprothese aus einem Oberschenkelteil 1 und einem Unterschenkelteil 2. Diese beiden Teile 1 und 2 werden mit dem Oberschenkel bzw. Unterschenkel des menschlichen Beines wirkverbunden. Auch kann das Unterschenkelteil 2 den Unterschenkel des menschlichen Beines ersetzen. Das Unterschenkelteil 2 und das Oberschenkelteil 1 sind durch ein Gelenk 3 miteinander verbunden. Hierbei handelt es sich um ein Gelenk, das als sogenannte dimere Gelenkkette ausgebildet ist. Dieses Gelenk besitzt zwei Gelenkachsen, und zwar die Gelenkachse M_{U} sowie die Gelenkachse M₀. Die Gelenkachse M_{U} ist dem Unterschenkelteil zugeordnet und die Gelenkachse M₀ ist dem Oberschenkelteil zugeordnet. Das Oberschenkelteil 1 weist innerhalb des Gelenkes 3 einen Gelenkfortsatz 4 auf. Dieser Gelenkfortsatz 4 besitzt eine Außenverzahnung 5. Das Unterschenkelteil 2 weist einen Gelenkabschnitt 6 auf, an dem eine Innenverzahnung 7 ausgebildet ist. Die Außenverzahnung 5 besitzt einen kurvenförmigen Verlauf, der dem Verlauf der Polkurve f des Oberschenkels im Funktionsbereich des Gelenks 3 entspricht. Die Innenverzahnung 7 besitzt einen kurvenförmigen Verlauf, der dem Verlauf der im Unterschenkel fixierten Polkurve t entspricht. Der Verlauf der Verzahnungen 5 und 7 kann exakt an den Verlauf der jeweiligen Polkurven bzw. Polbahnen angepaßt sein, so daß sich dann ein Kurvengetriebe ausbildet. Damit ist den Schenkelteilen 1, 2 und je eine Polbahn (je nach Bezugssystem eine Gang- bzw. Rastpolbahn) vorgegeben, die der physiologischen Bewegung des menschlichen Knies entspricht. In Streckstellung verläuft die Verzahnungskurve t des tibialen Gelenkteils 2 von kaudal nach kranial mit einer Ausbeulung nach posterior (Fig. 1). Die im Oberschenkelteil 1 fixierte Verzahnungskurve f hat einen von oben und von posterior her gesehen, konvexen Krümmungslauf. Da das Verzahnungskurvenpaar (bestehend aus der zum Unterschenkel 2 gehörenden Verzahnungskurve t und der zum Oberschenkel 1 gehörenden Verzahnungskurve f) nur bei Zugkräften zwischen Ober- und Unterschenkelteil in Kontakt bleibt, ist im Gelenk noch eine zweite gelenkige Verbindung (eine dimere Gelenkkette), bestehend aus einer im Oberschenkelteil 1 fixierten Achse M₀ und eine im Unterschenkelteil 2 fixierten Achse M₀ sowie deren Koppelglied 10 vorgesehen. Diese Verbindung sichert die Führungsfunktion des Verzahnungskurvenpaares auch bei kompressiven Kräften. Die im Oberschenkelteil 1 fixierte Achse M₀ schneidet die zum Oberschenkelteil 1 gehörende Verzahnungskurve f, die im Unterschenkelteil 2 fixierte Achse M_{U} schneidet die zum Unterschenkelteil 2 gehörende Verzahnungskurve t. Die Lagen der beiden Achsen sind so gewählt, daß der Abstand zwischen ihnen konstant bleibt, und daß damit so die Zwangführung durch das Verzahnungskurvenpaar nicht beeinflußt wird.

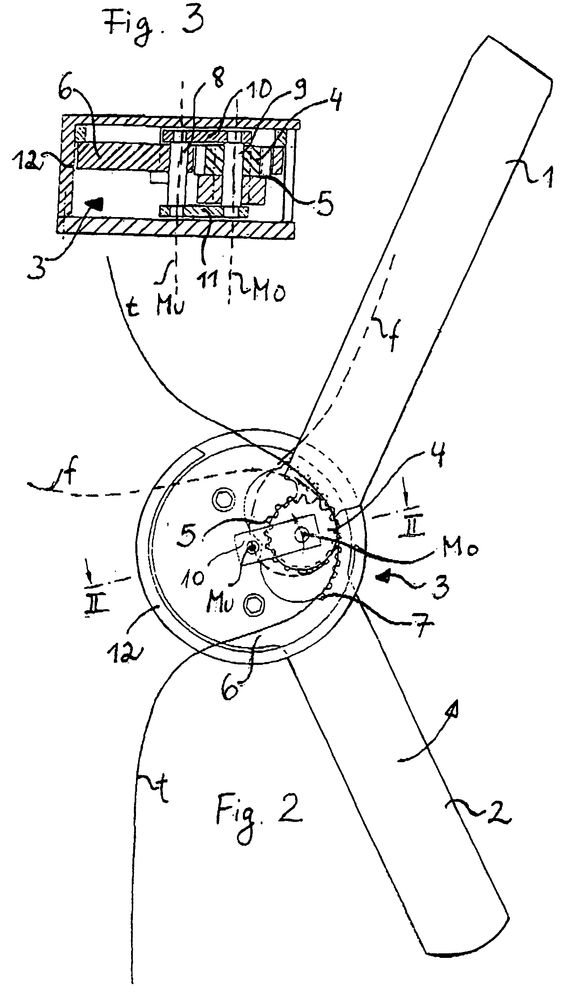

Im dargestellten Ausführungsbeispiel gemäß Fig. 2 sind die Verzahnungen 5, 7 Teil eines Kreisabschnittes, wobei die Kreisabschnitte jeweils Teile eines Kreises um die zugehörigen Gelenkachsen M₀ und M_{U} darstellen. Hierbei sind die Kreise im Gelenkfunktionsbereich dem Verlauf der jeweiligen Polkurven weitestgehend angenähert. Durch die Gelenkachsen M₀ und M_{U} sind, wie insbesondere aus Fig. 3 erkennbar, Lagerachsen 8, 9 hindurchgeführt. Die beiden Lagerachsen 8, 9 sind durch Koppelglieder 10, 11 miteinander gelenkig verbunden. Durch diese Koppelglieder 10, 11 ist wieder eine kraftübertragende dimere Kette innerhalb des Gelenks 3 ausgebildet, wobei ein Zwanglauf des Systems bei Krafteinwirkung garantiert wird. Die Koppelglieder 10, 11 verhindern ein Trennen der Verzahnungsbereiche während der Gelenkfunktion. Die Verzahnungen 5, 7 stehen in Wirkverbindung miteinander, so daß die beiden Verzahnungen bei einer Relativbewegung der Schenkelteile 1, 2 gegeneinander mit Ihren Abschnitten 4, 6 abrollen. Die Gelenkabschnitte 4, 6 sind innerhalb eines Gehäuses 12 angeordnet. Die Lagerachse 8 ist innerhalb des Gelenkabschnittes 6 geführt und die Lagerachse 9 ist innerhalb des Gelenkabschnittes 4 geführt.

Die erfindungsgemäße Exoprothese kann aus Kunststoff oder Metall oder aus einer Metall-Kunststoff-Kombination hergestellt werden.

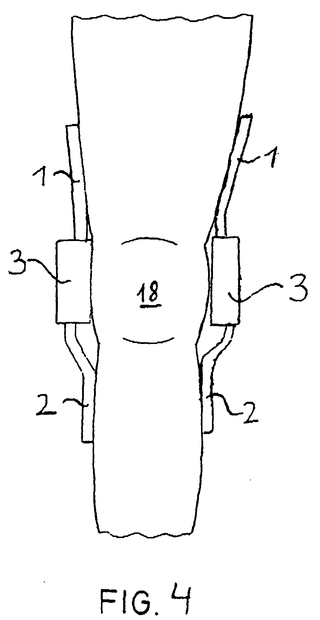

Wie aus Fig. 4 zu erkennen ist, wird die erfindungsgemäße Exoprothese ein- oder beidseitig, d.h. lateral und/oder medial am menschlichen Knie 18 befestigt.

Die vorliegende Erfindung ist nicht auf die gezeigten Ausführungsbeispiele beschränkt, sondern umfaßt alle in den Schutzbereich der Ansprüche fallenden Ausführungen.

## Patentansprüche

1. Exoprothese/Orthese für das menschliche Kniegelenk, bestehend aus einem mit dem menschlichen Oberschenkel verbindbaren Oberschenkelteil (1) und einem mit dem menschlichen Unterschenkel verbindbaren Unterschenkelteil (2), wobei die beiden Schenkelteile (1, 2) mindestens über ein Gelenk verbunden sind, das ein Gelenk mit zwei Gelenkachsen (M_{U}, M₀), d. h. eine sogenannte dimere Gelenkkette, bildet, die derart verbunden sind, daß sie eine Zwangbewegung durchführen, wobei den jeweiligen Schenkelteilen (1, 2) in bezug auf deren momentane Gelenkachse jeweils eine Polbahn zugeordnet ist, und am Oberschenkelteil (1) und am Unterschenkelteil (2) jeweils eine kurvenförmig verlaufende Verzahnung (5, 7) ausgebildet ist, die im Bewegungsfunktionsbereich des Gelenks (3) zahnradgetriebeartig zusammenwirken, wobei der Verlauf der Verzahnungskurven mit den Polbahnen (t, f) der beiden Schenkelteile (1, 2) im Bewegungsfunktionsbereich zusammenfallen, **dadurch gekennzeichnet, daß** in Streckstellung die Verzahnungskurve (t) des tibialen Gelenkteils (2) als Innenverzahnung (7) von kaudal nach kranial mit einer Ausbeulung nach posterior verläuft und die im femuralen Gelenkteil (1) fixierte Verzahnungskurve (f) als Außenverzahnung (5) einen nach kaudal und von posterior her gesehen konvexen Krümmungsverlauf aufweist.

2. Exoprothese nach Anspruch 1,
**dadurch gekennzeichnet, daß** die Verzahnungen auf einem Kreisabschnitt eines Kreises liegen, dessen Mittelpunkt jeweils mit der zugeordneten Gelenkachse (M₀ ,M_{U} ) übereinstimmt.

3. Exoprothese nach einem der Ansprüche 1 oder 2,
**dadurch gekennzeichnet, daß** die beiden Gelenkabschnitte (4, 6) der Schenkelteile (1, 2) durch mindestens ein Koppelglied (10, 11) zwischen ihren Gelenkachsen (8, 9) gelenkig verbunden sind.

4. Exoprothese nach einem der Ansprüche 1 bis 3,
**gekennzeichnet durch**, ein zweites, in einer parallelen Ebene angeordnetes Medialgelenk (11) bzw. Lateralgelenk.

## Claims

1. Exoprosthesis/orthosis for a human knee-joint, consisting of an upper leg part (1), which may be connected to a human upper leg, and a lower leg part (2), which may be connected to a human lower leg, the two leg parts (1, 2) being connected at least via a joint, which forms a joint with two joint axes (M_{U}, M_{O}), i.e. a so-called dimeric joint chain, which are connected in such a way that they carry out a forced movement, a respective polode being associated with each of the respective leg parts (1, 2), with respect to its momentary joint axis, and curved teething (5, 7), which cooperates in the manner of toothed gearing in the movement functional region of the joint (3), being configured on the upper leg part (1) and on the lower leg part (2), the course of the teething curves coinciding with the polodes (t, f) of the two leg parts (1, 2) in the movement functional region, **characterised in that**, in a stretched position, the teething curve (t) of the tibial leg part (2) extends posteriorly with a bulge, as the inner teething (7) from the caudal region to the cranial region, and the teething curve (f) that is fixed in the femoral leg part (1), as the outer teething (5), has a curvature that extends toward the cranial region and that, viewed posteriorly, is convex.

2. Exoprosthesis according to claim 1, **characterised in that** the teething is located on a circular segment of a circle, the centre of which corresponds to the respective associated joint axis (M_{O}, M_{U}).

3. Exoprosthesis according to any one of claims 1 and 2, **characterised in that** the two joint sections (4, 6) of the leg parts (1, 2) are connected in an articulated manner between their joint axes (8, 9) by at least one coupling member (10, 11).

4. Exoprosthesis according to any one of claims 1 to 3, **characterised by** a second, medial joint (11) or lateral joint that is arranged in a parallel plane.

## Revendications

1. Exoprothèse/orthèse pour le genou humain, consistant en une partie de cuisse (1) pouvant être reliée à la cuisse humaine et en une partie de mollet (2) pouvant être reliée au mollet humain, sachant que les deux parties de jambe (1, 2) sont au moins reliées par une articulation, laquelle forme une articulation à deux axes articulés (Mᵤ, Mₒ), c'est-à-dire une chaîne articulée plus souple, les axes articulés étant reliés de telle sorte qu'ils effectuent un mouvement forcé, sachant qu'une polhodie est respectivement associée aux parties de jambe (1, 2) par rapport à leur axe articulé momentané, et qu'un engrènement (5, 7) s'étendant en forme de courbure est respectivement configuré au niveau de la partie de cuisse (1) et au niveau de la partie de mollet (2), ledit engrènement entrant en interaction comme un engrenage dans la zone de la fonction de la locomotion de l'articulation (3), sachant que l'allure des courbures d'engrènement coïncide avec les polhodies (t, f) des deux parties de jambe (1, 2) dans la zone de la fonction de la locomotion, **caractérisée en ce que** dans la position étendue, la courbure d'engrènement (t) de la partie articulée (2) tibiale s'étend comme un engrènement intérieur (7) allant de la zone caudale vers la zone crânienne avec une bosse vers l'arrière et que la courbure d'engrènement (f) fixée dans la partie articulée (1) fémorale présente comme engrènement extérieur (5) une courbure convexe vue vers la zone caudale et de l'arrière.

2. Exoprothèse selon la revendication 1, **caractérisée en ce que** les engrènements reposent sur un segment de cercle d'un cercle dont le centre coïncide respectivement avec l'axe articulé (Mᵤ, Mₒ) associé.

3. Exoprothèse selon l'une quelconque des revendications 1 ou 2, **caractérisée en ce que** les deux segments articulés (4, 6) des parties de jambe (1, 2) sont reliés de manière articulée entre leurs axes articulés (8, 9) par au moins un élément de couplage (10, 11).

4. Exoprothèse selon l'une quelconque des revendications 1 à 3, **caractérisée par** une deuxième articulation médiale (11) ou articulation latérale disposée dans un plan parallèle.
